(19) Europäisches Patentamt
European Patent Office
Office européen des brevets

(11) Veröffentlichungsnummer : **0 110 235**
**B1**

(12) # EUROPÄISCHE PATENTSCHRIFT

(45) Veröffentlichungstag der Patentschrift : **04.03.87**

(51) Int. Cl.⁴ : **C 07 C102/06**, C 07 C103/76

(21) Anmeldenummer : **83111428.5**

(22) Anmeldetag : **15.11.83**

(54) **Verfahren zur Herstellung von Terephthalsäure-monoamid-Derivaten.**

(30) Priorität : **22.11.82 DE 3243148**

(43) Veröffentlichungstag der Anmeldung : **13.06.84 Patentblatt 84/24**

(45) Bekanntmachung des Hinweises auf die Patenterteilung : **04.03.87 Patentblatt 87/10**

(84) Benannte Vertragsstaaten : **AT BE CH DE FR GB IT LI NL SE**

(56) Entgegenhaltungen :
**FR-A- 926 115**
**FR-A- 1 365 574**
**US-A- 2 798 087**
**US-A- 3 288 794**
**CHEMICAL ABSTRACTS, Band 61, Nr. 13, 21. Dezember 1964, Spalte 16020e, Columbus, Ohio, USA**
**CHEMICAL ABSTRACTS, Band 84, 1976, Seite 426, Nr. 30699m, Columbus, Ohio, USA**

(73) Patentinhaber : **HOECHST AKTIENGESELLSCHAFT**
**Postfach 80 03 20**
**D-6230 Frankfurt am Main 80 (DE)**

(72) Erfinder : **Ritschel, Werner, Dr.**
**Gartenstrasse 17**
**D-6238 Hofheim am Taunus (DE)**

EP 0 110 235 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es ist bekannt, daß Terephthalsäure-halbamide in Form ihrer Salze Öle und organische Lösemittel gelieren können. Solche Gele werden als Schmiermittel eingesetzt. Diese Verbindungen konnten bisher in genügender Reinheit nur nach einem aufwendigen, mehrstufigen Verfahren hergestellt werden, bei dem man zunächst aus Dimethylterephthalat das Alkali-halbsalz herstellt und dieses dann über die Stufe des Säurechlorids in das Halbamid überführt (Ind. and Eng. Chem. *49*, (1957) 1691). Nachteilig bei diesem Verfahren ist nicht nur ein hoher Verbrauch an Lösemittel, sondern auch die Verwendung einer Hilfsbase bei der Umsetzung der Säurechlorids mit dem Amin. Dies wiederum bedeutet eine hohe Kostenbelastung und führt zu Problemen bei der Filtration des Hydrochlorids.

Aus der US-A 3 288 794 ist die Herstellung von Dicarbonsäureamiden aus Dicarbonsäurediestern und Aminen in Gegenwart von Alkalialkoholat als Katalysator bekannt. In den Fällen, bei denen dort als Amide die Carbonsäureester-halbamide mit anfallen, ist aber die Ausbeute sehr gering. In gleicher Weise ist in der FR-A 926 115 ganz allgemein die Herstellung von Dicarbonsäureamiden durch Umsetzung von Dicarbonsäurediestern mit Aminen in Gegenwart von Alkalialkoholaten erwähnt. Die Herstellung speziell von Terephthalsäuremonoester-halbamiden ist beschrieben in Chem. Abstr. Bd 61 (1964) 16020e. Die dort beschriebene Reaktion verläuft allerdings bei relativ hohen Temperaturen (140 bis 170 °C). Auch in Chem. Abstr. Bd 84 (1976) 30699 m ist die Herstellung dieser Terephthalsäuremonoester-halbamide beschrieben. Dieses Verfahren hat aber den Nachteil, daß dabei ein großer stöchiometrischer Überschuß an Terephthalsäurediester als Ausgangsprodukt notwendig ist.

Es stellte sich daher die Aufgabe, ausgehend von Dimethylterephthalat ein vereinfachtes Verfahren zur Herstellung solcher Terephthalsäure-halbamide bzw. deren Methylester zu finden.

Gegenstand der Erfindung ist ein Verfahren zur Herstellung von Terephthalsäuremonoamid-Derivaten der Formel

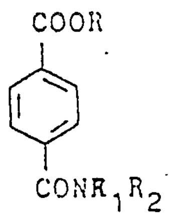

worin R Wasserstoff, ein Alkali- oder Erdalkalimetall-Kation oder Methyl, $R_1$ $C_8$-$C_{30}$-Alkyl, vorzugsweise $C_{12}$-$C_{22}$-Alkyl und $R_2$ $C_1$-$C_{30}$-Alkyl und vorzugsweise Wasserstoff bedeutet, wobei man 1 Mol Dimethylterephthalat in Gegenwart von Natriummethylat mit einem Mol eines Amins der Formel

$$HNR_1R_2$$

in siedendem Methanol umsetzt.

Wesentlich für das erfindungsgemäße Verfahren ist die Verwendung von Methanol als Lösemittel und die Anwesenheit von Natriummethylat als Katalysator. Da das Verhältnis von Lösemittel zu den Reaktionspartnern einen besonderen Einfluß hat auf Verhältnis von Halbamid zu Diamid im Endprodukt, empfiehlt es sich nur soviel Methanol zu nehmen, daß die Reaktionsmischung nach der Auskristallisation des Halbamids gerade noch gut rührbar bleibt. Nimmt man zuviel Methanol, wird mehr Terephthalsäurediamid als Nebenprodukt gebildet. Die Menge an Katalysator hat einen Einfluß auf die Reaktionszeit. Bevorzugt arbeitet man mit einer Menge von 0,1 bis 1 Mol Natriummethylat. Die Reaktionszeiten betragen ca. 4 Stunden bei 1 Mol Natriummethylat und ca. 8 Stunden bei 0,5 Mol Natriummethylat. Die Reaktion wird bei der Siedetemperatur des Methanols durchgeführt. Bedingt durch die Stöchiometrie der Reaktionsgleichung arbeitet man vorzugsweise mit einem Molverhältnis 1 : 1 der beiden Ausgangsverbindungen.

Nach Beendigung der Reaktion wird das ausgefallene Produkt in der üblichen Weise abfiltriert, gewaschen und getrocknet. Wie die folgenden Beispiele zeigen, enthält das Reaktionsprodukt in Abhängigkeit von der Länge der Alkylgruppen in dem Amin noch mehr oder weniger große Anteile des Terephthalsäure-diamids. Diese Diamide lassen sich von den Halbamiden durch die üblichen Verfahren der fraktionierten Kristallisation trennen, falls dies gewünscht ist. Für die erwähnte Verdickung von Ölen und Lösemittel reichen aber die erfindungsgemäß erhältlichen Gemische aus Halbamid und Diamid völlig aus. Auch ist es für diesen Einsatzzweck nicht erforderlich, den zunächst bei der beschriebenen Reaktion anfallenden Methylester separat zu verseifen. Man kann vielmehr diesen Ester direkt dem Öl bzw. dem Lösemittel zugeben und dann durch Zugabe von Alkali zum Alkalisalz verseifen. Voraussetzung hierfür ist selbstverständlich, daß das Öl bzw. das Lösemittel selbst nicht verseift werden kann. Auf der anderen Seite kann natürlich der Methylester auch separat nach bekannten Verfahren verseift werden zum Alkalisalz. Aus dem Alkalisalz lassen sich dann nach ebenfalls bekannten Verfahren die feie Säure und

Salze mit anderen Kationen gewinnen.

Beispiel 1

In 150 ml Methanol werden 19,4 g (0,1 Mol) Dimethylterephthalat und 27 g (0,1 Mol) Octadecylamin unter Rückfluß erhitzt und dann werden 18 g (0,1 Mol) Natriummethylat (30 %ig in Methanol) rasch zugetropft. Man beobachtet nach der Zugabe von $NaOCH_3$ zunächst eine vollständige Auflösung des Dimethylterephthalats, worauf binnen kurzem das Produkt auskristallisiert. Man rührt noch 4 Stunden unter Rückfluß, kühlt auf Raumtemperatur ab und saugt den Niederschlag ab. Man wäscht dann zunächst mit Methanol und dann mit Wasser, bis das Filtrat neutral reagiert.

Man erhält 36 g (83,3 %) 4-Carbomethoxy-benzoesäure-octadecylamid mit einem Gehalt von 11 % an Terephthalsäure-bis-octadecylamid.

Anstelle von 18 g Natriummethylat kann man auch nur 9 g (0,05 Mol) einsetzen, muß dann aber insgesamt 8 Stunden unter Rückfluß erhitzen. Man erhält ein Reaktionsprodukt gleicher Zusammensetzung und gleicher Qualität wie oben.

Beispiel 2

19,4 g (0,1 Mol) Dimethylterephthalat und 18,5 g (0,1 Mol) Dodecylamin werden in 100 ml Methanol unter Rückfluß erhitzt. Nach Zufügen von 9 g (0,05 Mol) Natriummethylat (30 %ig in Methanol) erhitzt man noch 8 Stunden unter Rückfluß und arbeitet den Reaktionsansatz auf wie in Biepiel 1 angegeben.

Man erhält 31,7 g (91,2 %) 4-Carbomethoxy-benzoesäure-dodecylamid mit einem Gehalt von 14 % an Terephthalsäure-bis-dodecylamid.

Beispiel 3

19,4 g (0,1 Mol) Dimethylterephthalat und 12,9 g (0,1 Mol) Octylamin werden in 100 ml Methanol mit 9 g (0,05 Mol) Natriummethylat (30 %ig) 8 Stunden unter Rückfluß erhitzt. Nach der Aufarbeitung wie in Beispiel 1 angegeben erhält man 26,5 g (91 %) 4-Carbomethoxy-benzoesäure-octylamid mit einem Gehalt von 30 % an Terephthalsäure-bis-octylamid.

Der Gehalt an Terephthalsäure-bisamid wurde in allen Fällen durch $^1H$-NMR Spektroskopie ermittelt.

## Patentanspruch

Verfahren zur Herstellung von Terephthalsäure-monoamid-Derivaten der Formel

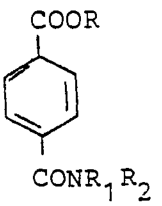

worin R Wasserstoff, ein Erdalkali- oder Alkalimetall-Kation oder Methyl, $R_1$ $C_8$-$C_{30}$-Alkyl, und $R_2$ Wasserstoff oder $C_1$-$C_{30}$-Alkyl bedeutet, dadurch gekennzeichnet, daß man 1 Mol Dimethylterephthalat in Gegenwart von Natriummethylat mit einem Mol eines Amins der Formel

$$HNR_1R_2$$

in siedendem Methanol umsetzt.

## Claim

A process for preparing terephthalic acid monoamide derivatives of the formula

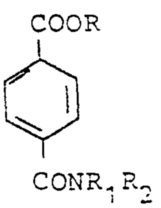

in which R denotes hydrogen, an alkaline earth metal or alkali metal cation or methyl, $R_1$ denotes $C_8$-$C_{30}$-alkyl, and $R_2$ denotes hydrogen or $C_1$-$C_{30}$-alkyl, which comprises reacting one mole of dimethyl terephthalate with one mole of an amine of the formula

$$HNR_1R_2$$

in boiling methanol and in the presence of sodium methylate.

**Revendication**

Procédé de préparation de dérivés d'acides téréphtalamiques qui répondent à la formule suivante :

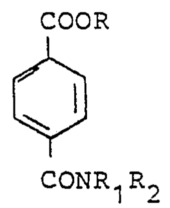

dans laquelle R représente l'hydrogène, un cation de métal alcalin ou de métal alcalino-terreux ou un radical méthyle, $R_1$ représente un radical alkyle en $C_8$-$C_{30}$, et $R_2$ représente l'hydrogène ou un radical alkyle en $C_1$-$C_{30}$, procédé caractérisé en ce que l'on fait réagir 1 mol de téréphtalate de diméthyle, en présence de méthylate de sodium, avec 1 mol d'une amine de formule :

$$HNR_1R_2,$$

dans un méthanol bouillant.